(19) Europäisches Patentamt
European Patent Office
Office européen des brevets

(11) **EP 4 382 114 A1**

(12) **EUROPEAN PATENT APPLICATION**

(43) Date of publication:
**12.06.2024 Bulletin 2024/24**

(21) Application number: **23213930.3**

(22) Date of filing: **04.12.2023**

(51) International Patent Classification (IPC):
*A61K 36/53* (2006.01)     *A61K 36/185* (2006.01)
*A61K 31/015* (2006.01)     *A61K 31/07* (2006.01)
*A61K 31/355* (2006.01)     *A61K 31/593* (2006.01)
*A61P 29/00* (2006.01)     *A61P 31/00* (2006.01)

(52) Cooperative Patent Classification (CPC):
(C-Sets available)
**A61P 31/00; A61K 31/015; A61K 31/07;
A61K 31/355; A61K 31/593; A61K 36/185;
A61K 36/53; A61P 29/00**     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.12.2022 IT 202200025059**

(71) Applicant: **Green Farm Lab S.r.l.
29122 Piacenza (IT)**

(72) Inventors:
• **GIOIA, Fabio
29122 Piacenza (IT)**
• **PAGANI, Gian Luca
29122 Piacenza (IT)**
• **PETROSINO, Paolo
29122 Piacenza (IT)**

(74) Representative: **Delbarba, Andrea
Bugnion S.p.A.
Viale Lancetti, 17
20158 Milano (IT)**

(54) **DIETETIC FEED WITH STIMULATING FUNCTION**

(57)     The present invention relates to a composition comprising a plant belonging to the genus Ocimum or a portion thereof and/or an extract of a plant belonging to the genus Ocimum, at least one sesquiterpene, at least one vitamin and a plant or a portion thereof belonging to the genus Moringa. Furthermore, the invention relates to the use of the composition in the treatment or prevention of a mastitis in a dairy animal.

Fig. 1

**(Cont. next page)**

(52) Cooperative Patent Classification (CPC): (Cont.)

C-Sets
**A61K 31/015, A61K 2300/00;**
**A61K 31/07, A61K 2300/00;**
**A61K 31/355, A61K 2300/00;**
**A61K 31/593, A61K 2300/00;**
**A61K 36/185, A61K 2300/00;**
**A61K 36/53, A61K 2300/00**

**Description**

**FIELD OF THE INVENTION**

The present invention relates to a composition and the use thereof in the treatment or prevention of a mastitis in a dairy animal.

**STATE OF THE ART**

Mastitis is an inflammation of the mammary gland that causes changes in the biochemical composition of milk and in the gland tissue.

It is one of the most common diseases in dairy cattle or cows. Mastitis has a negative impact on the quality and quantity of milk produced, so much so as to cause losses for the farmer. If the pathology is not treated in time, it can become chronic, making it necessary to sacrifice the animal.

Mastitis is a multifactorial disease, as the infection depends on the germs, environmental conditions, and characteristics of the cow. Microorganisms invade the mammary tissue, causing an inflammation of the gland.

The disease has two main aetiologies: contagious mastitis, caused by microorganisms that live in the mammary gland (mainly *Streptococcus agalactiae* and *Staphylococcus aureus*) and environmental mastitis, caused by microorganisms (environmental streptococci and coliforms) that live in the environment and are transmitted between milking and the dry period during which the gland does not produce milk.

According to the symptoms, mastitis is classified as:

- subclinical mastitis, where no alterations in the milk and/or the udders are observed, but the quantity of microorganisms and somatic cells is high;
- clinical mastitis, with inflammation and pain for the animal, altered milk which shows flakes, clots, a watery appearance and sometimes blood; and
- acute mastitis, which places the animal's life at risk, with fever, loss of appetite or lower milk production.

One of the parameters that is most widely assessed, and is an indicator of incipient mastitis, is the somatic cell count in milk. In fact, an increase in the somatic cell count in milk is indicative of an inflammation of the mammary gland: more than 200,000 cells/ml of milk are indicative of subclinical mastitis.

The current standard treatment for mastitis provides for the use of antibiotics. The milk must thus be discarded for 15 days and, besides the risk of contamination of the food chain and the possible development of antibiotic resistance, there is also the suffering of the infected cows. Regulation (EU) 2019/6 on veterinary medicinal products was promulgated in January 2020 and fully implemented as of January of this year. The document, highly elaborate, replaces the legislation currently in force and poses some major limits to the use of drugs, above all antibiotics. The provisions contained in Regulation (EU) 2019/6, along with the growing pressure of public opinion for an overall reduction of antibiotic treatments in livestock, give rise to objective difficulties in the use of antibiotics for controlling mastitis and make it even more important to adopt protocols that significantly improve hygiene on farms and, above all, stimulate the search for substances capable of increasing nonspecific immune defences, in the absence of vaccines of proven effectiveness in field conditions.

Thus, there is a need to have a treatment which enables the animal's health to be restored while avoiding production losses for the farmer.

**SUMMARY OF THE INVENTION**

A first aspect of the present invention relates to a composition comprising a plant belonging to the genus Ocimum or a portion thereof and/or an extract of a plant belonging to the genus Ocimum, at least one sesquiterpene, at least one vitamin and a plant or a portion thereof belonging to the genus Moringa.

Preferibilmente, the plant belonging to the genus Ocimum belongs to the species *O.sanctum.* Said plant belonging to the genus Ocimum is preferably present in the composition in a concentration of between 70 and 98% weight/weight (w/w) relative to the weight of the composition, preferably between 80 and 96% w/w, even more preferably between 85 and 95% w/w. Preferably, the extract of the plant belonging to the genus Ocimum is present in a concentration of between 0.3 and 1.5% volume/weight (v/w) relative to the weight of the composition, preferably between 0.4 and 1.2% v/w, even more preferably between 0.5 and 1% v/w.

The at least one sesquiterpene is preferably present in a concentration of between 0.3 and 1.5% volume/weight (v/w) relative to the weight of the composition, preferably between 0.4 and 1.2% v/w, even more preferably between 0.5 and 1% v/w.

Preferably, the at least one vitamin is selected from vitamin A, vitamin D and vitamin E. Said at least one vitamin is preferably present in a concentration of between 0.2 and 1.5% weight/weight (w/w) relative to the weight of the composition, more preferably between 0.3 and 1.2% w/w, even more preferably between 0.4 and 1% w/w.

The at least one plant belonging to the genus Moringa is preferably present in a concentration of between 5 and 13% weight/weight (w/w) relative to the weight of the composition, preferably between 6 and 12% w/w, even more preferably between 7 and 10% w/w.

The above-described composition is preferably formulated as a powder, preferably in the form of a dried or dehydrated powder.

A second aspect of the present invention relates to the composition as described above for use in the prevention or treatment of a mastitis in a dairy animal. Preferably, the somatic cell count in the milk obtained from the dairy animal is less than $2*10^5$ cells/ml of milk or the somatic cell count in the milk obtained from the dairy animal is greater than $2*10^5$ cells/ml of milk, preferably greater than $2.5*10^5$ cells/ml of milk.

## BRIEF DESCRIPTION OF THE FIGURES

Figure 1 shows a graph with the total somatic cell concentrations in the milk of healthy bovines and bovines with subclinical mastitis, before, during and after treatment with the claimed composition;

Figure 2 shows a graph with the total somatic cell concentrations in the milk of bovines with subclinical mastitis, before, during and after treatment with the claimed composition;

Figure 3 shows a graph with the total somatic cell concentrations in the milk of healthy bovines, before, during and after treatment with the claimed composition;

Figure 4 shows a graph with the somatic cell concentrations per ml of milk of healthy bovines and bovines with subclinical mastitis, before, during and after treatment with the claimed composition;

Figure 5 shows a graph with the somatic cell concentrations per ml of milk of bovines with subclinical mastitis, before, during and after treatment with the claimed composition;

Figure 6 shows a graph with the somatic cell concentrations per ml of milk of healthy bovines, before, during and after treatment with the claimed composition;

Figure 7 shows a graph with the number of milkings per day for healthy bovines and bovines with subclinical mastitis, before, during and after treatment with the claimed composition;

Figure 8 shows a graph with the number of milkings per day for bovines with subclinical mastitis, before, during and after treatment with the claimed composition;

Figure 9 shows a graph with the number of milkings per day for healthy bovines, before, during and after treatment with the claimed composition;

Figure 10 shows a graph with the percentage concentrations of polymorphonuclear neutrophil leukocytes and lymphocytes in the milk of healthy bovines and bovines with subclinical mastitis, before, during and after treatment with the claimed composition;

Figure 11 shows a graph with the percentage concentrations of polymorphonuclear neutrophil leukocytes and lymphocytes in the milk of bovines with subclinical mastitis, before, during and after treatment with the claimed composition;

Figure 12 shows a graph with the percentage concentrations of polymorphonuclear neutrophil leukocytes and lymphocytes in the milk of healthy bovines, before, during and after treatment with the claimed composition;

Figure 13 shows a graph with the variations, compared to before treatment, in the percentage concentrations of polymorphonuclear neutrophil leukocytes and lymphocytes in the milk of bovines with subclinical mastitis, during and after treatment with the claimed composition;

Figure 14 shows a graph with the variations, compared to before treatment, in the percentage concentrations of polymorphonuclear neutrophil leukocytes and lymphocytes in the milk of healthy bovines, during and after treatment with the claimed composition;

Figure 15 shows a graph with the percentage concentrations of polymorphonuclear neutrophil leukocytes and lymphocytes per ml of milk of healthy bovines and bovines with subclinical mastitis, before, during and after treatment with the claimed composition;

Figure 16 shows a graph with the percentage concentrations of polymorphonuclear neutrophil leukocytes and lymphocytes per ml of milk of bovines with subclinical mastitis, before, during and after treatment with the claimed composition;

Figure 17 shows a graph with the percentage concentrations of polymorphonuclear neutrophil leukocytes and lymphocytes per ml of milk of healthy bovines, before, during and after treatment with the claimed composition;

Figure 18 shows a graph with the variations, compared to before treatment, in the percentage concentrations of polymorphonuclear neutrophil leukocytes and lymphocytes per ml of milk of bovines with subclinical mastitis during and after treatment with the claimed composition;

Figure 19 shows a graph with the variations, compared to before treatment, in the percentage concentrations of polymorphonuclear neutrophil leukocytes and lymphocytes per ml of milk of healthy bovines during and after treatment with the claimed composition;

Figure 20 shows a graph with the percentage concentrations of polymorphonuclear neutrophil leukocytes and lymphocytes in the milk of an entire day of healthy bovines and bovines with subclinical mastitis before, during and after treatment with the claimed composition;

Figure 21 shows a graph with the percentage concentrations of polymorphonuclear neutrophil leukocytes and lymphocytes in the milk of an entire day of bovines with subclinical mastitis before, during and after treatment with the claimed composition;

Figure 22 shows a graph with the percentage concentrations of polymorphonuclear neutrophil leukocytes and lymphocytes in the milk of an entire day of healthy bovines before, during and after treatment with the claimed composition;

Figure 23 shows a graph with the variations, compared to before treatment, in the percentage concentrations of polymorphonuclear neutrophil leukocytes and lymphocytes in the milk of bovines with subclinical mastitis produced over an entire day during and after treatment with the claimed composition;

Figure 24 shows a graph with the variations, compared to before treatment, in the percentage concentrations of polymorphonuclear neutrophil leukocytes and lymphocytes in the milk of healthy bovines produced over an entire day during and after treatment with the claimed composition;

Figure 25 shows a graph with the amount of milk produced daily by healthy bovines and bovines with subclinical mastitis before, during and after treatment with the claimed composition;

Figure 26 shows a graph with the amount of milk produced daily by bovines with subclinical mastitis before, during and after treatment with the claimed composition;

Figure 27 shows a graph with the amount of milk produced daily by healthy bovines before, during and after treatment with the claimed composition;

Figure 28 shows a graph with the percentage content of fat in the milk of healthy bovines and bovines with subclinical mastitis before, during and after treatment with the claimed composition;

Figure 29 shows a graph with the percentage content of fat in the milk of bovines with subclinical mastitis before, during and after treatment with the claimed composition;

Figure 30 shows a graph with the percentage content of fat in the milk of healthy bovines before, during and after treatment with the claimed composition;

Figure 31 shows a graph with the percentage content of proteins in the milk of healthy bovines and bovines with subclinical mastitis before, during and after treatment with the claimed composition;

Figure 32 shows a graph with the percentage content of proteins in the milk of bovines with subclinical mastitis before, during and after treatment with the claimed composition;

Figure 33 shows a graph with the percentage content of proteins in the milk of healthy bovines before, during and after treatment with the claimed composition;

Figure 34 shows a graph with the percentage content of caseins in the milk of healthy bovines and bovines with subclinical mastitis before, during and after treatment with the claimed composition;

Figure 35 shows a graph with the percentage content of caseins in the milk of bovines with subclinical mastitis before, during and after treatment with the claimed composition;

Figure 36 shows a graph with the percentage content of caseins in the milk of healthy bovines before, during and after treatment with the claimed composition.

**DEFINITIONS**

In the context of the present invention, the term "Ocimum" means an annual herbaceous plant, belonging to the family Lamiaceae.

In the context of the present invention, the expression "*Ocimum sanctum*" means a plant also known as "*Ocimum tenuiflorum*" "holy basil", "Tulasī ", and "Tulsi".

**DETAILED DESCRIPTION OF THE INVENTION**

In the present invention, where not specified otherwise, the concentrations refer to the weight of the composition.

A first aspect of the present invention relates to a composition comprising a plant belonging to the genus Ocimum or a portion thereof and/or an extract of a plant belonging to the genus Ocimum, at least one sesquiterpene, at least one vitamin and a plant or a portion thereof belonging to the genus Moringa.

In one embodiment, the plant belonging to the genus Ocimum belongs to the species *O.sanctum.* Preferably, the plant belonging to the species *O.sanctum* belongs to at least one variety selected from *Krishna, Shri* or *Ram tulsi* and *Vana tulsi.* In a preferred embodiment, the plant belonging to the species *O.sanctum* belongs to the *Krishna* variety.

In one embodiment, the plant belonging to the genus Ocimum is present in a concentration of between 70 and 98% weight/weight (w/w) relative to the weight of the composition, preferably between 80 and 96% w/w, even more preferably between 85 and 95% w/w.

Preferably, the plant belonging to the genus Ocimum is at least a portion of said plant; it is preferably at least one leaf and/or at least one flower of Ocimum. In one embodiment, the plant belonging to the genus Ocimum is in dried or dehydrated form.

In one embodiment, the plant belonging to the genus Ocimum has a total polyphenol content of between 5500 and 7000 mg/Kg, preferably between 6000 and 6800 mg/Kg.

In one embodiment, the plant belonging to the genus Ocimum has a total polysaccharide content of between 10 and 35 g/100 g, preferably between 15 and 25 g/100 g.

In one embodiment, the plant belonging to the genus Ocimum has a caryophyllene content of between 2 and 5 mg/Kg, preferably between 3 and 4 mg/Kg.

In one embodiment, the extract of the plant belonging to the genus Ocimum is present in a concentration of between 0.3 and 1.5% volume/weight (v/w) relative to the weight of the composition, preferably between 0.4 and 1.2% v/w, even more preferably between 0.5 and 1% v/w.

In one embodiment, the extract of the at least one plant belonging to the genus Ocimum has an estragole content of between $2*10^4$ and $7*10^4$ mg/Kg, preferably between $3.5*10^4$ and $5.5*10^4$ mg/Kg relative to the weight of the extract of the at least one plant belonging to the genus Ocimum.

In one embodiment, the extract of the at least one plant belonging to the genus Ocimum has a 3-Carene content of between $1.5*10^4$ and $3.5*10^4$ mg/Kg, preferably between $2*10^4$ and $3*10^4$ mg/Kg relative to the weight of the extract of the at least one plant belonging to the genus Ocimum. Preferably, the extract of the at least one plant belonging to the genus Ocimum is obtained by distillation of at least one portion of said plant, preferably by distillation of at least one leaf and/or at least one flower of Ocimum. Preferably, said extract is obtained by steam distillation of at least one leaf and/or at least one flower of the plant belonging to the genus Ocimum. Preferably, said extract is an essential oil of the plant belonging to the genus Ocimum; more preferably, it is an essential oil of *O.sanctum.*

In one embodiment, the at least one sesquiterpene is preferably a selective agonist of the cannabinoid type 2 receptor (CB2); more preferably, it is β-caryophyllene.

In one embodiment, the at least one sesquiterpene is present in a concentration of between 0.3 and 1.5% volume/weight (v/w) relative to the weight of the composition, preferably between 0.4 and 1.2% v/w, even more preferably between 0.5 and 1% v/w.

In a preferred embodiment of the invention, the extract of the plant belonging to the genus Ocimum and the at least one sesquiterpene are dispersed in a carrier, more preferably an oil, even more preferably in an oil selected from sunflower oil, linseed oil, and olive oil. In one embodiment, the carrier is sunflower oil.

Preferably, said at least one vitamin is selected from vitamin A, vitamin D and vitamin E.

In one embodiment, the composition comprises at least two vitamins selected from vitamin A, vitamin D, and vitamin E. Preferably, the composition comprises vitamin A, vitamin D, and vitamin E.

In one embodiment, the at least one vitamin is preferably an encapsulated vitamin, more preferably a micro-encapsulated vitamin. Said at least one vitamin is preferably encapsulated in a carrier in order to increase the stability and bioavailability of said at least one vitamin. Said carrier is a carrier known to the person skilled in the art. Said carrier is for example a phospholipid vesicle, preferably a liposome or a micelle.

In one embodiment, the at least one vitamin is present in a concentration of between 0.2 and 1.5% weight/weight (w/w) relative to the weight of the composition, preferably between 0.3 and 1.2 % w/w, even more preferably between 0.4 and 1 % w/w.

In one embodiment, the plant belonging to the genus Moringa preferably belongs to the species *M.olifera.* Preferably, the composition comprises at least one portion of the plant belonging to the genus Moringa; the composition preferably comprises at least one leaf and/or at least one fruit and/or at least one flower and/or at least one root of said plant. In one embodiment, the plant belonging to the genus Moringa is in dried or dehydrated form.

In one embodiment, the at least one plant belonging to the genus Moringa is present in a concentration of between 5 and 13% weight/weight (w/w) relative to the weight of the composition, preferably between 6 and 12% w/w, even more preferably between 7 and 10% w/w.

In a preferred embodiment of the invention, the composition comprises or consists of a plant or a part thereof and an extract of a plant belonging to the genus Ocimum, at least one sesquiterpene, vitamins A, D and E, and a plant belonging to the genus Moringa.

The composition of the present invention can further comprise one or more emulsifying agents, and/or pH stabilisers, and/or preservatives, and/or other ingredients known to the person skilled in the art.

In one embodiment, the above-described composition is formulated as a powder, preferably in the form of a dried or dehydrated powder.

In a further embodiment, the above-described composition is formulated as a solution or emulsion, or as a dispersion.

Preferably, the composition is prepared by mixing together the above-mentioned ingredients with techniques known to the person skilled in the art. Preferably, the plant belonging to the genus Ocimum is mixed with the at least one vitamin and the plant belonging to the genus Moringa and then the extract of a plant belonging to the genus Ocimum and the at least one sesquiterpene are added. Preferably, the extract of the plant belonging to the genus Ocimum and the at least one sesquiterpene are dispersed in a carrier, more preferably an oil, even more preferably in an oil selected from sunflower oil, linseed oil, olive oil. In one embodiment, the carrier is sunflower oil.

In one embodiment, the mixture comprises the extract of a plant belonging to the genus Ocimum and β-caryophyllene in an Ocimum extract:β-caryophyllene ratio of between 1:2 and 2:1, preferably in a 1:1 ratio.

In a preferred embodiment, the composition is dried or dehydrated with techniques known to the person skilled in the art.

A second aspect of the present invention relates to a composition as described above in detail for use in the prevention or treatment of a mastitis in a dairy animal.

In one embodiment, the dairy animal is a bovine, preferably a dairy cow or buffalo, or it is an ovine, preferably a sheep or a goat. In a preferred embodiment of the invention, the dairy animal is a cow or a buffalo.

In one embodiment, the animal is in normal, i.e., non-pathological conditions of health. In this case, the above-described composition is used or administered to prevent the occurrence of clinical, subclinical, or acute mastitis in the animal.

Preferably, the somatic cell count in the milk is less than $2*10^5$ cells/ml of milk.

In one embodiment, the mastitis is a subclinical mastitis, i.e., there are no evident modifications as regards the mammary gland or the milk. The subclinical mastitis is preferably characterised by a reduction in the amount of milk produced and an increase in the somatic cell count in the milk compared to the somatic cell count in normal, i.e., non-pathological conditions. Preferably, the somatic cell count in the milk is greater than $2*10^5$ cells/ml of milk, preferably greater than $2.5*10^5$ cells/ml of milk.

In one embodiment, the mastitis is a clinical mastitis, i.e., characterised by evident modifications of the mammary gland and of the milk. The clinical mastitis is preferably characterised by an increase in the somatic cell count in the milk compared to the somatic cell count in normal, i.e., non-pathological conditions.

Preferably, the somatic cell count in the milk is greater than $2.5*10^5$ cells/ml of milk, preferably greater than $3*10^5$ cells/ml of milk.

In a further embodiment, the mastitis is an acute mastitis, i.e., characterised by fever, pain, a decrease in milk production, and an increase in somatic cells.

In a further embodiment, the mastitis is caused by at least one pathogenic agent; said pathogenic agent is preferably a virus or a bacterium or a fungus. Preferably, said at least one pathogenic agent is a bacterium, more preferably it is a bacterium selected from: a mastitogenic Streptococcus, preferably *Streptococcus uberis, Streptococcus dygalactiae, Streptococcus agalactiae*, *Staphylococcus aureus* and *Escherichia Coli.*

In one embodiment, the composition is administered at least once a day, preferably at least twice a day. Preferably, the composition is administered by mixing it with the feed administered to the animal.

In other words, the composition is administered twice a day with the feed that is normally administered to the animal. For the medical uses described above, the composition is preferably administered twice a day, corresponding to an amount of the plant belonging to the genus Ocimum of between 70 and 100 g per meal/animal, preferably between 80 and 90 g per meal/animal, and corresponding to an amount of the plant belonging to the genus Moringa of between 4 and 10 g per meal/animal, preferably between 5 and 9 g per meal/animal.

Preferably, the above-described composition is administered for at least 6 days, more preferably for at least 10 successive days.

The Applicant has surprisingly discovered that administering the composition of the present invention prevents or reduces the effects of a mastitis, preferably of a subclinical mastitis in a dairy animal. In particular, the composition of the present invention is capable of modulating the immune response, by increasing the total number of polymorphonuclear cells (PMNs) and lymphocytes in the milk, which represent the main nonspecific cell defences of the udder, without giving negative effects on the quality of the milk or the characteristics of the milk itself. Such effects, as shown in the example, are very marked in the treatment period and also remain, albeit to a less evident degree, in the period after the treatment.

A third aspect of the present invention relates to a method for the treatment or prevention of a mastitis in a dairy animal. Said method comprises at least a step of administering a composition as described above to a dairy animal. In one embodiment, the dairy animal is a bovine, preferably a dairy cow, or an ovine, preferably a sheep or a goat. In a preferred embodiment of the invention, the dairy animal is a cow.

In one embodiment, the animal is in normal, i.e., non-pathological conditions of health. In this case, the above-described composition is used to prevent the occurrence of clinical or subclinical mastitis in the animal.

Preferably, the somatic cell count in the milk is less than $2*10^5$ cells/ml of milk.

Preferably, the mastitis is a subclinical mastitis, i.e. there are no evident modifications as regards the mammary gland or the milk. The subclinical mastitis is preferably characterised by a reduction in the amount of milk produced and an increase in the somatic cell count in the milk compared to the somatic cell count in normal, i.e., non-pathological conditions. Preferably, the somatic cell count in the milk is greater than $2*10^5$ cells/ml of milk, preferably greater than $2.5*10^5$ cells/ml of milk.

Preferably, the mastitis is a clinical mastitis, i.e., characterised by evident modifications of the mammary gland and of the milk. The clinical mastitis is preferably characterised by an increase in the somatic cell count in the milk compared to the somatic cell count in normal, i.e., non-pathological conditions.

Preferably, the somatic cell count in the milk is greater than $2.5*10^5$ cells/ml of milk, preferably greater than $3*10^5$ cells/ml of milk.

In a further embodiment, the mastitis is an acute mastitis, i.e., characterised by fever, pain, a decrease in milk production and an increase in somatic cells.

In a further embodiment, the mastitis is caused by at least one pathogenic agent; said pathogenic agent is preferably a bacterium or a fungus. Preferably, said at least one pathogenic agent is a bacterium, more preferably it is a bacterium selected from: a mastitogenic Streptococcus, preferably *Streptococcus uberis, Streptococcus dygalactiae, Streptococcus agalactiae*, *Staphylococcus aureus* and *Escherichia Coli.*

**EXAMPLE**

1. Farm, sampling and analysis

The field trial was conducted on a farm with a number of about 360 lactating cows milked with a robotic milking system (5 units and 3 groups of bovines). Samples were collected before the start of treatment (01/02/2022), at the end of treatment (17/02/2022), and one month after the first sampling (01/03/2022).

The collection of milk samples and evaluation of daily production were carried out on all lactating bovines by means of an automated system connected to 5 different milking robots according to the procedures provided for by the Associazione Italiana Allevatori (Italian Breeders Association). The samples were analysed with a combined FOSS 7/dc instrument which enables an evaluation both of qualitative parameters (e.g., fat, proteins) and of total and differential cell parameters according to standards ISO 13366 and IDF 148.

2. Treatment

The product was administered with the ration normally used at the farm, at a dose of 85.4 g per meal (170.8 g per day) for 10 days for a total of 1708 g per head.

The product was added to a mixer wagon that was used solely for the purpose of providing the ration containing the supplement. The control bovines received the same diet, less the supplement, mixed in a different wagon.

3. Database and statistical analyses

The data obtained from the analyses performed in the three sampling sessions mentioned earlier were associated with the weekly reports produced by the management software of the milking robots on the same dates as the sampling in a single database and subsequently analysed by the author.

In particular, the analysis of data regarding the effectiveness of the treatment was carried out with the following model:

var response = treatment (yes/no) + presence of subclinical mastitis (yes/no) + days of lactation (classes of 30 days) + number of lactations (1,2,3, >3) + period of treatment (pre-, during, post-) + all the interactions of the treatment with other factors + error.

The bovine was considered as a random factor in the model.

For the statistical assessments, a value of $\alpha$=0.05 was considered.

4. Results

4.1 Samples

For the statistical analyses, a total number of 818 observations were considered. This number comprises only the bovines that were always in the same group (treated or control) and for at least 2 tests out of 3. Therefore, the bovines that changed group and/or had less than 2 tests were not considered. In practical terms, 491 samples were considered in the control group, while 327 samples were included in the treated group. The distribution of samples in the 3 tests provided for is shown in table 1, from which one observes a substantial constancy in the samples collected on the three planned dates and no statistically significant differences were detected.

**Table 1**

|  | Pre- | End | 1 month |
|---|---|---|---|
| **Control** | 160 (60.38%) | 167 (59.85%) | 164 (59.85%) |
| **Treated** | 105 (39.62%) | 112 (40.15%) | 110 (40.15%) |
| **Total** | 265 | 279 | 274 |

Table 2 shows the distribution of treatments based on the age of the animals.

**Table 2**

|  | Number of lactations | | | | Total |
|---|---|---|---|---|---|
|  | **1** | **2** | **3** | **>3** |  |
| **Control** | 111 | 151 | 127 | 102 | **491** |
| **Treated** | 111 | 89 | 58 | 69 | **327** |
| **Total** | **222** | **240** | **185** | **171** | **818** |

The distribution demonstrates that there is a significant difference in the composition of the groups based on the number of lactations (table 3). This table shows the significance of the difference between the expected frequency and the observed frequency. As may be observed, we have a proportion of bovines in second lactation that is less than expected and greater for the third in the treated group, and the opposite in the control group.

**Table 3**

| Lactations | | | | |
|---|---|---|---|---|
|  | 1 | 2 | 3 | >3 |
| Control | > | > | < | < |
| Treated | < | < | > | > |

Table 4, on the other hand, shows the distributions of samples based on the days of lactation. In this case as well, significant differences are observed in the periods of 31-90 days and 241-300 days.

**Table 4**

**Days of Lactation**

| | 1-30 | 31-60 | 61-90 | 91-120 | 121-150 | 151-180 | 181-210 | 211-140 | 241-270 | 271-300 | Total |
|---|---|---|---|---|---|---|---|---|---|---|---|
| **24 h control** | 24 | 63 | 55 | 61 | 80 | 67 | 46 | 31 | 17 | 11 | **491** |
| **Treated** | 7 | 24 | 21 | 31 | 41 | 48 | 34 | 25 | 27 | 26 | **327** |
| **Total** | 31 | 87 | 76 | 92 | 123 | 115 | 80 | 56 | 44 | 37 | **818** |

4.2 Result of the treatment

The means recorded for all the variables considered are shown in table 5, whereas the results of the statistical analyses as described in the Materials & Methods section are shown in table 6.

**Table 5**

| Parameter | | Control | | Treated | |
|---|---|---|---|---|---|
| | Unit of measurement | Mean | Std. Dev. | Mean | Std. Dev. |
| Total SSC | $Log_{10}$ | 9.40 | 0.61 | 9.50 | 0.65 |
| SSC/ml | $Log_{10}$/ml | 4.75 | 0.63 | 4.88 | 0.67 |
| No. of milkings/day | N | 2.75 | 0.73 | 3.01 | 0.69 |
| DSCC | % | 61.35 | 17.06 | 66.29 | 16.17 |
| PMN+LYM/ml | $Log_{10}$/ml | 4.52 | 0.72 | 4.69 | 0.75 |
| Tot. PMN+LYM | $Log_{10}$ | 9.17 | 0.70 | 9.30 | 0.73 |
| Milk | Kg/day | 45.59 | 11.19 | 42.68 | 11.08 |
| Fat | % | 3.67 | 0.76 | 3.84 | 0.78 |
| Proteins | % | 3.27 | 0.35 | 3.40 | 0.37 |
| Caseins | % | 2.57 | 0.28 | 2.68 | 0.31 |

The data show that there are significantly higher means in the treated group as regards the immune parameters and in particular DSCC, total PMN+LYM/ml and total PMN+LYM. Significant differences with higher values in the treated group were also found for SCC/ml, mean number of milkings and protein and casein content.

In the graphs below, all the factors considered are analysed, broken down by period of treatment and based on state of health observed in the pre-treatment period, bovines with ≤200,000 SCC/ml being defined as healthy, and bovines defined as having subclinical mastitis when the SCC/ml value exceeded that threshold.

**Table 6**

4.2.1 Total somatic cells

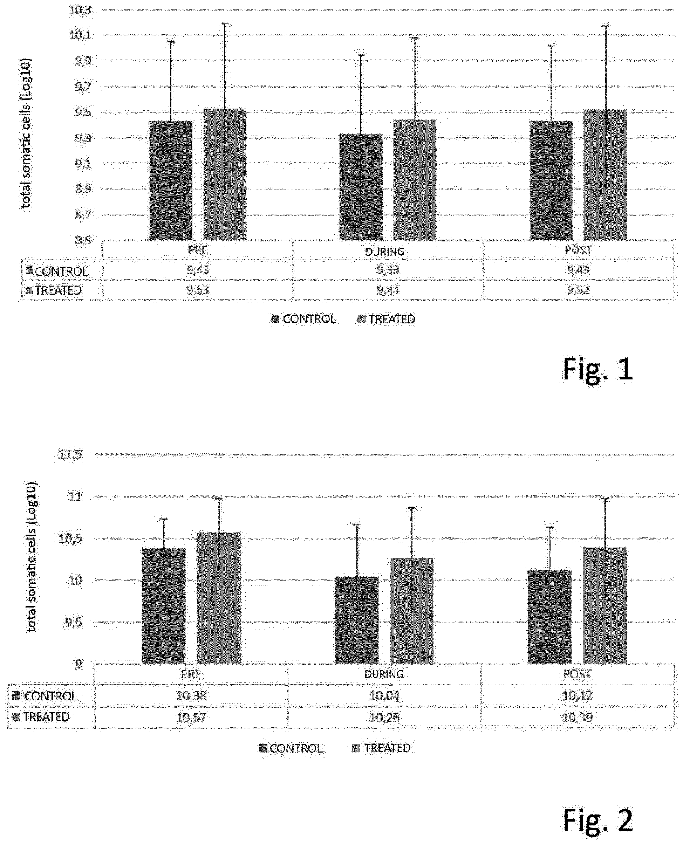

As shown in figure 1, the data relating to the set of analyses broken down by period of treatment show a similar trend in the treated group and in control group, with higher means in the treated group.

| Factor | SCC/M L | TOT SCC | MEAN MILK. | DSCC | PMN+L YM/ML | TOT PMN+L YM | MILK (KG) | FAT (%) | PROT (%) | CASEI NS (%) |
|---|---|---|---|---|---|---|---|---|---|---|
| Treatme nt | 0.042 | | 0.008 | 0.0010 | 0.041 | 0.036 | | | 0.044 | 0.042 |
| Days of lactat. | 0.035 | | <0.001 | | | | <0.001 | <0.001 | <0.001 | <0.001 |
| No. of lactat. | | 0.006 | | | | 0.015 | <0.001 | | <0.001 | <0.001 |
| Subclini cal mastitis | <0.001 | | <0.001 | <0.001 | <0.001 | <0.001 | <0.001 | | | |
| Treatme nt period | | 0.004 | | 0.001 | | 0.014 | <0.001 | 0.034 | <0.001 | <0.001 |
| Treat. days of lactat. | | | | | | | | | | |
| Treat. No. of lactat. | | | | | | | 0.018 | | | |
| Treat. Subcl. Mast. | | | | | | | | | | |
| Treat. Period of treat. | | | | 0.006 | | | 0.023 | | | 0.046 |

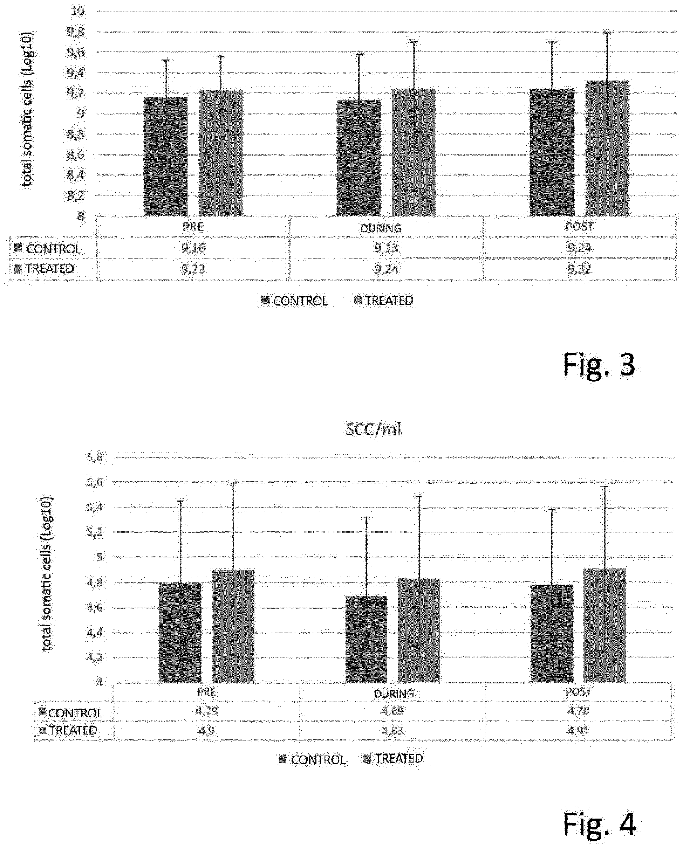

When the data were analysed based on state of health, it was possible to observe a decrease in the values during the treatment period for both groups in the bovines with subclinical mastitis (Figure 2). In the case of healthy bovines, by contrast, a constant increase was observed in the treated group, whereas there was a slight decrease in the control group during the treatment period (Figure 3). The data suggest that the treatment has an effect of stimulating the cell response, a response that is more evident in healthy bovines, whereas in bovines that already have an inflammatory cell response (subclinical mastitis), there is not this effect, which, if present, would be considered negative, but rather a slight reduction, confirming that the product has an immunomodulating effect on the nonspecific immune response.

4.2.2 Somatic cells/ml

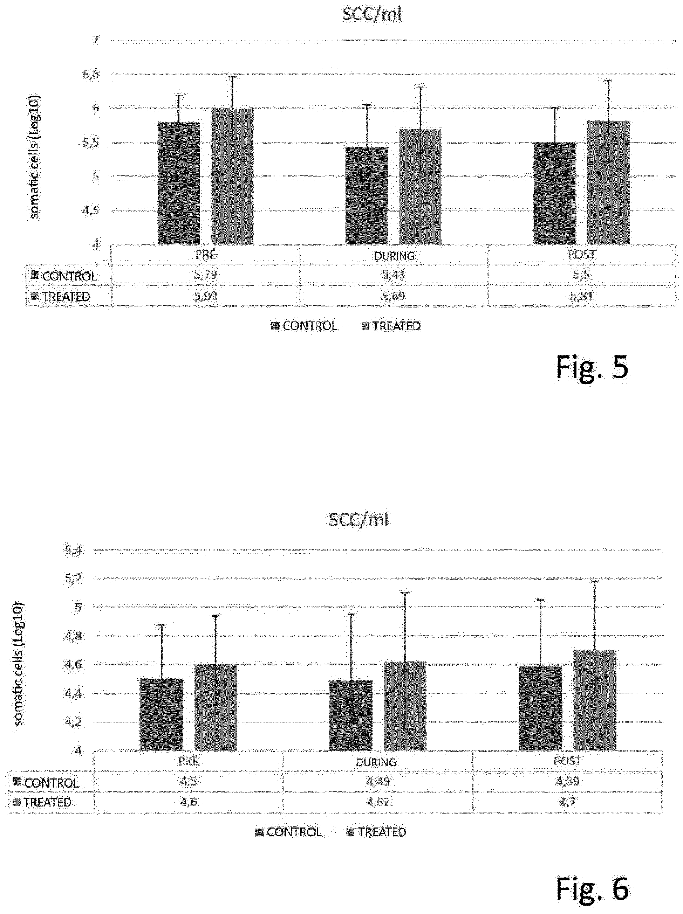

As shown in figure 4, the data relating to the set of analyses broken down by period of treatment show a slight decrease in the control group in the treatment period, a decrease that is much smaller in the treated group and a value in the post-treatment period that is higher for the treated group and lower for the control group, compared to the pre-treatment period. When the data were analysed based on state of health, it was possible to observe a decrease in values during the treatment period for both groups in the case of bovines with subclinical mastitis (Figure 5). In the case of healthy bovines, by contrast, one observes a constant increase in the treated group, whereas there is a slight decrease in the control group during the treatment period (Figure 6). These data confirm the immunomodulating effect already observed for the total cell count.

4.2.3 Mean number of milkings per day

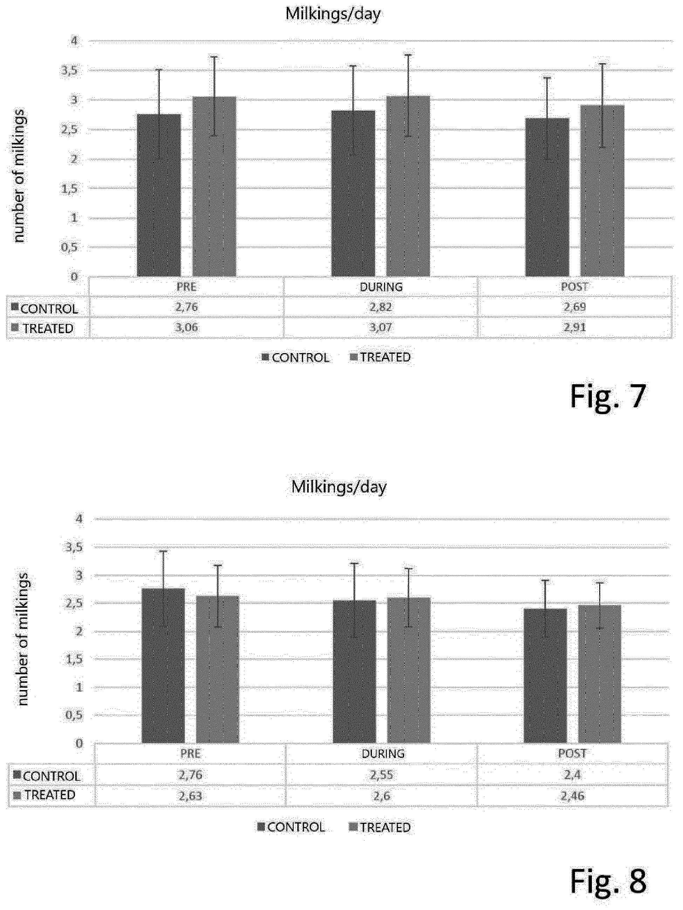

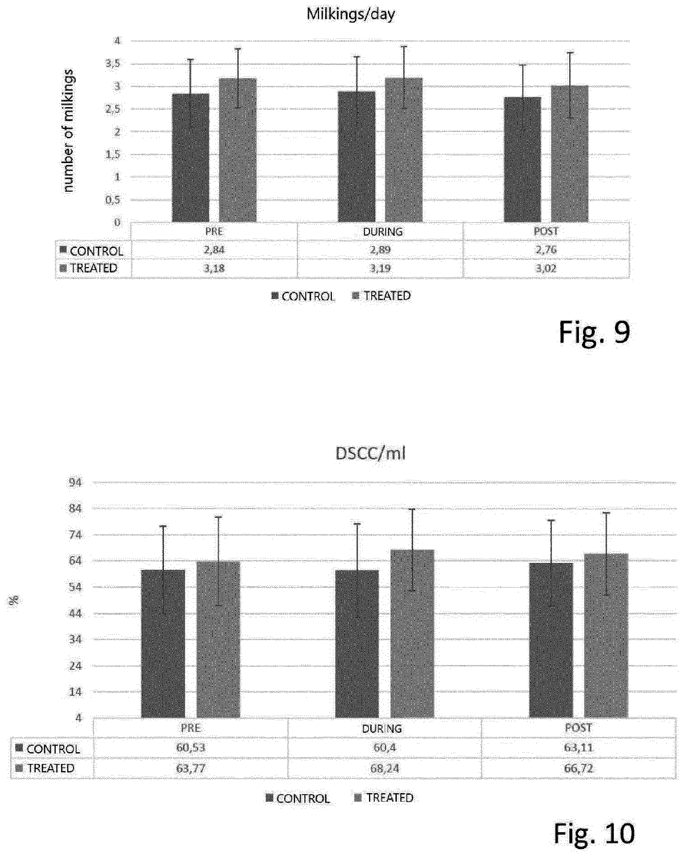

As shown in Figure 7, the data relating to the set of analyses broken down by period of treatment show decidedly higher values in the treated group compared to the control group. The values for both groups, however, fell in the follow-up period, as expected, given the increase in lactations. When the data were analysed based on state of health, it was possible to observe a progressive decrease in the values in both groups, both in bovines with subclinical mastitis (Figure 8) and in healthy bovines (Figure 9), even though this reduction occurred only in the post-treatment period in the treated group and during treatment in the control group.

4.2.4 Proportion of polymorphonuclear neutrophil (PMN) leukocytes and lymphocytes

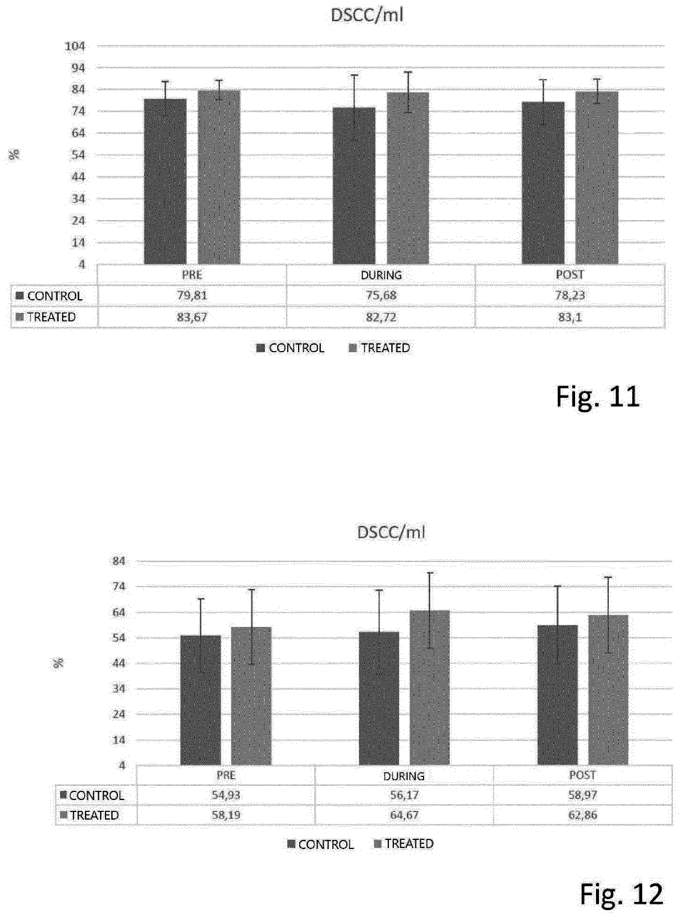

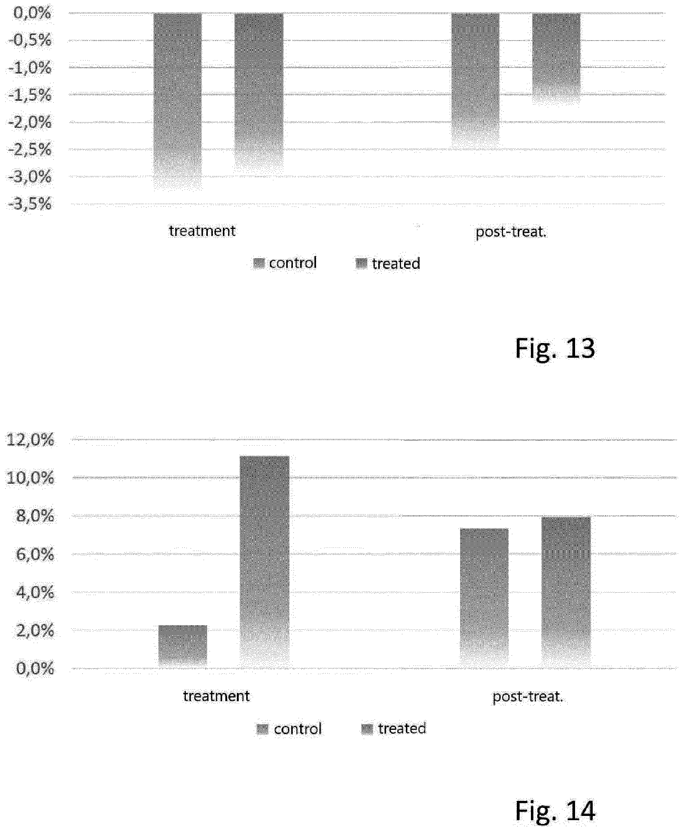

As shown in Figure 10, the data relating to the set of analyses broken down by period of treatment show a similar trend in the treated and control groups, with higher means in the treated group compared to the control group. It may be observed, moreover, that in the treatment period the values increased decidedly in the treated group, but remained stable in the control group, only increasing in the post-treatment period. When the data were analysed based on state of health, it was possible to observe a decrease in the values during the treatment period in both groups for the bovines with subclinical mastitis (Figures 11 and 13) and a more marked decrease in the case of the control group. In the case of healthy bovines, by contrast, a marked increase was observed in the treated group during the treatment period, with values that were substantially stable in the post-treatment period as well (Figures 12 and 14). This trend suggests an immunomodulating effect with minimal variations in the case of animals with an ongoing inflammatory process and an immunostimulating one instead in healthy subjects.

4.2.5 Amount of PMNs + lymphocytes in milk per ml

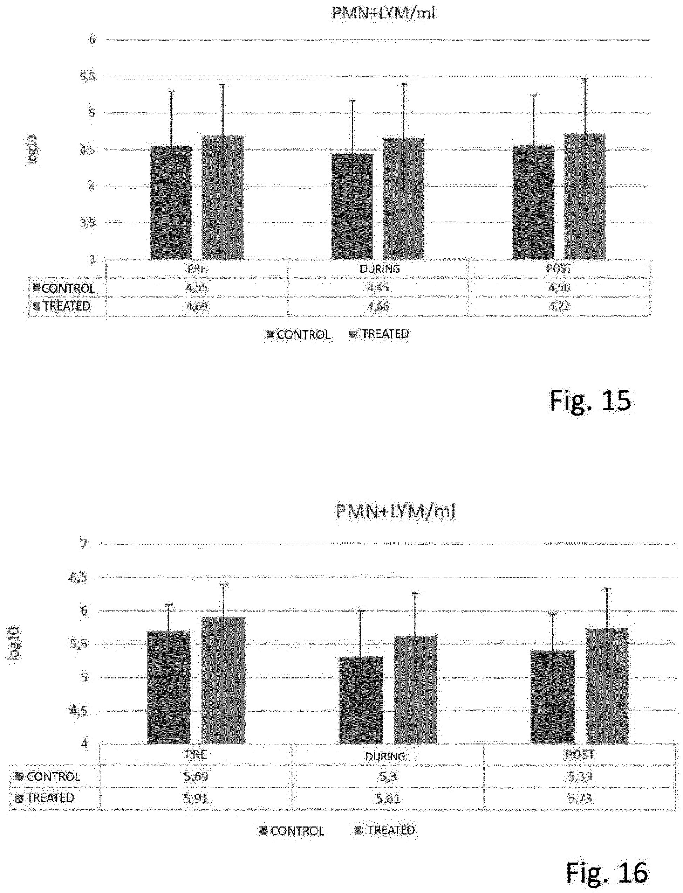

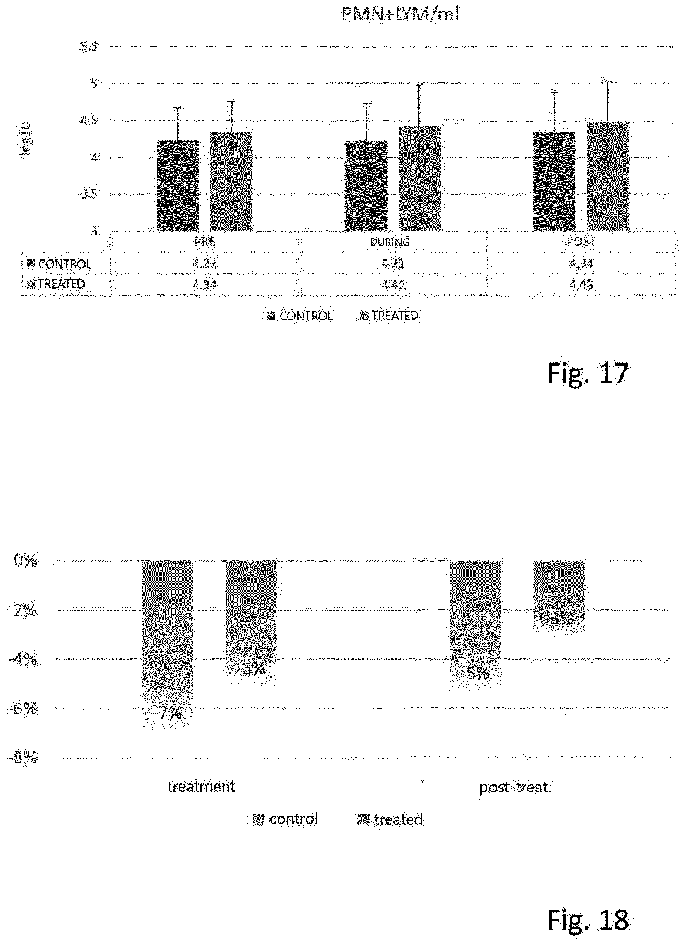

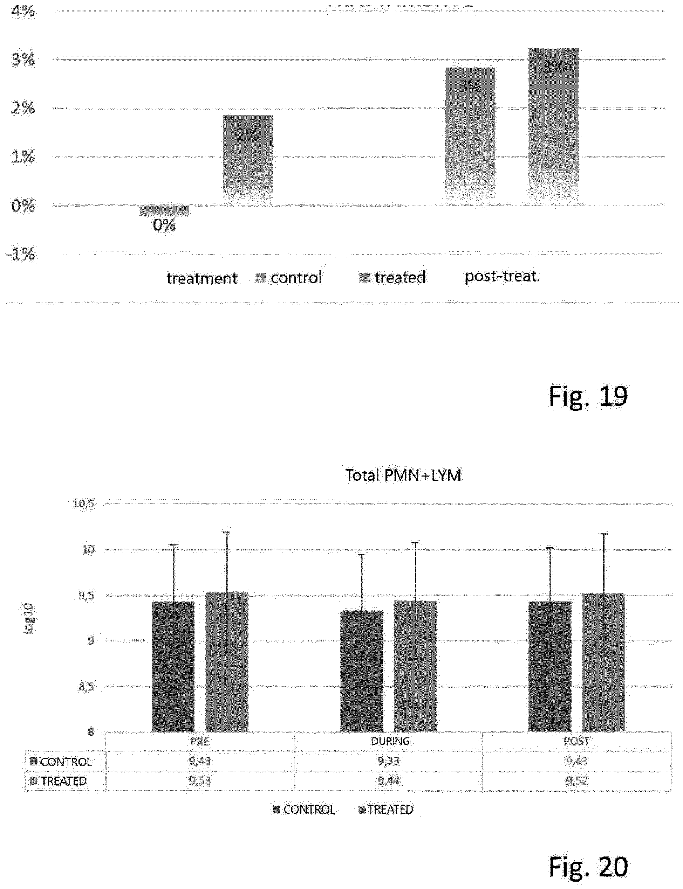

As shown in Figure 15, the data relating to the set of analyses broken down by period of treatment show a similar trend in the treated and control groups, with higher means in the treated group compared to the control group, but with a constant trend in both groups. When the data were analysed based on state of health, it was possible to observe a decrease in the values during the treatment period for both groups of bovines with subclinical mastitis (Figures 16 and 18). In the case of healthy bovines, by contrast, a marked increase was observed in the treated group during the treatment period, with values substantially on the rise also in the post-treatment period, whereas the control group showed an increase only in the post-treatment period (Figures 17 and 19).

4.2.6 Total amount of PMNs + lymphocytes in milk produced over an entire day

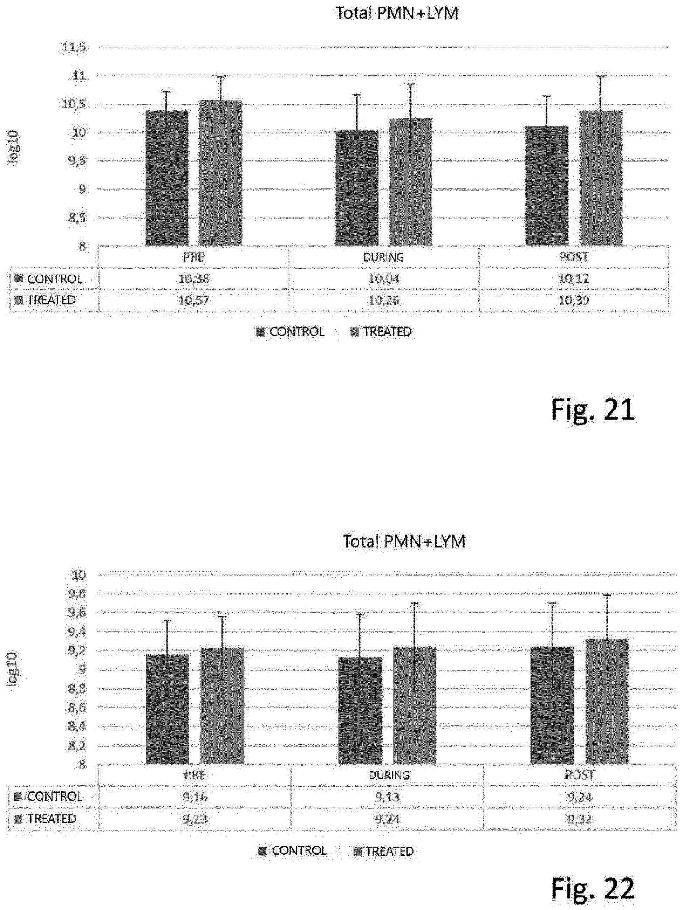

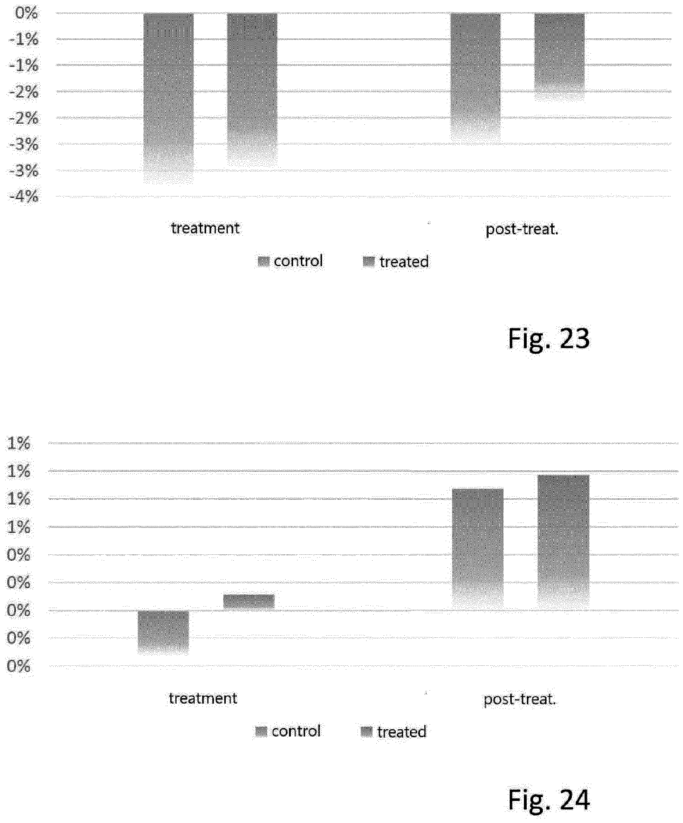

As shown in figure 20, the data relating to the set of analyses broken down by period of treatment show a similar trend in the treated and control groups, with higher means in the treated group compared to the control group, but with a parallel and small reduction during the treatment period in both groups. When the data were analysed based on state of health, it was possible to observe a decrease in the values during the treatment period for both groups of bovines with subclinical mastitis (Figures 21 and 23). In the case of healthy bovines, by contrast, a progressive increase was observed in the treated group, whereas the control group showed a small decrease in the post-treatment period (Figure 22 and 24).

### 4.2.7 Amount of milk produced daily

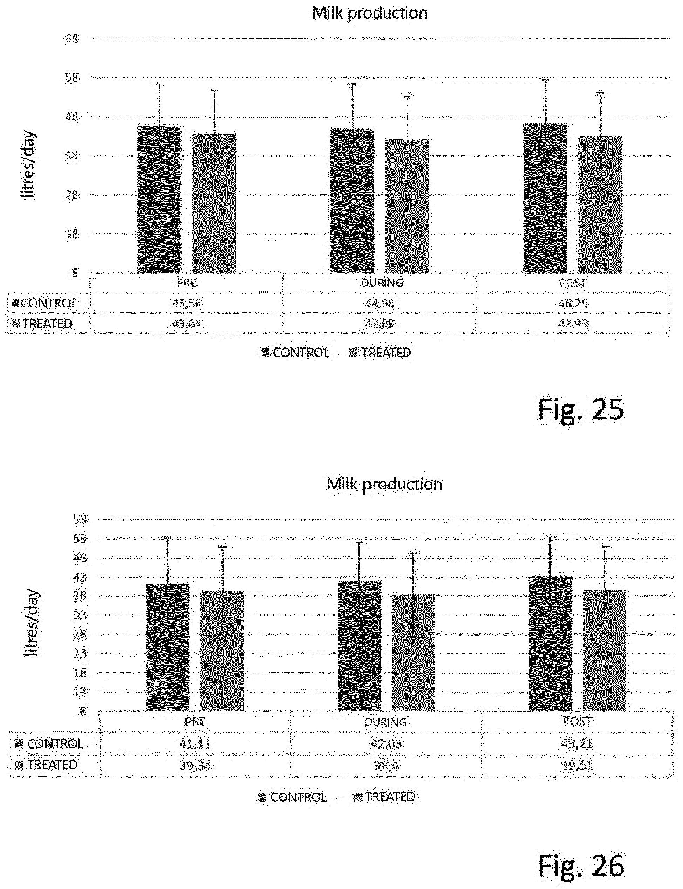

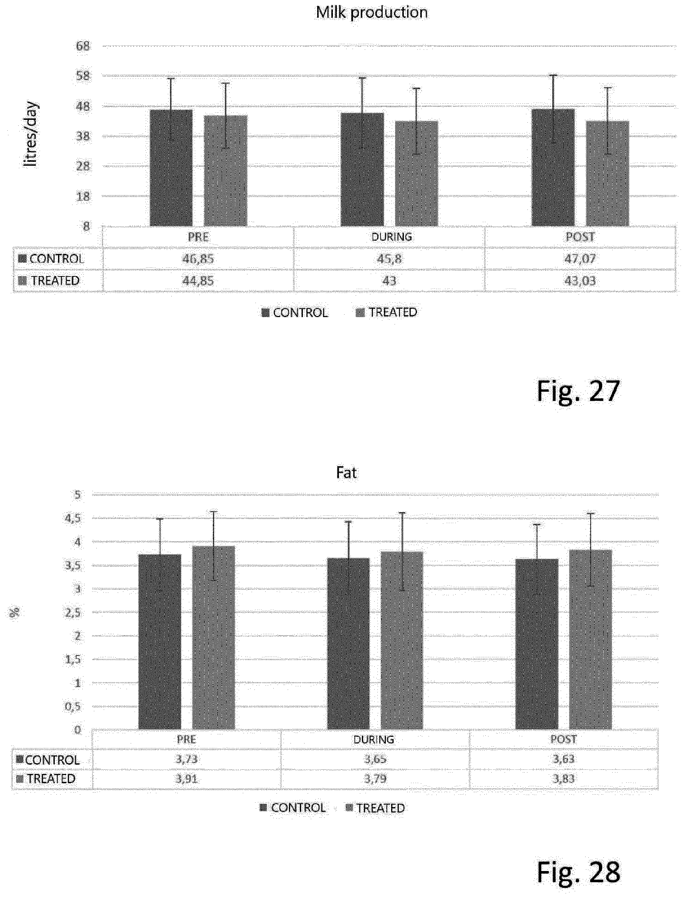

As shown in figure 25, the data relating to the set of analyses broken down by period of treatment show a similar trend in the treated and control groups, with lower means in the treated group compared to the control group. In both groups, the values were progressively reduced, as expected, due to the increase in the days of lactation. When the data were analysed based on state of health, it was possible to observe a similar increase for the control group, whereas the values remained substantially constant in the treated group (Figure 26). In the case of healthy bovines, by contrast, a progressive reduction was observed in both groups (Figure 27).

### 4.2.8 Percentage content of fat

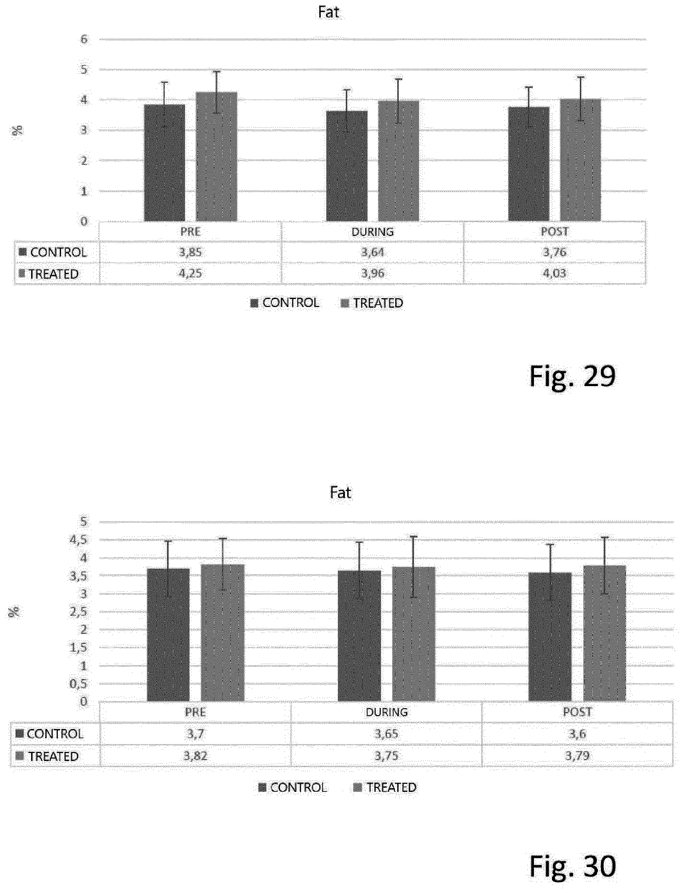

As shown in figure 28, the data relating to the set of analyses broken down by period of treatment show a similar trend in the treated and control groups, with higher means in the treated group compared to the control group. For both groups, the values increased progressively, as expected, given the increase in the days of lactation. When the data were analysed based on state of health, it was possible to observe a trend wholly similar to the general one as regards both the presence or absence of treatment and the presence or absence of subclinical mastitis (Figures 29 and 30).

### 4.2.9 Percentage content of proteins

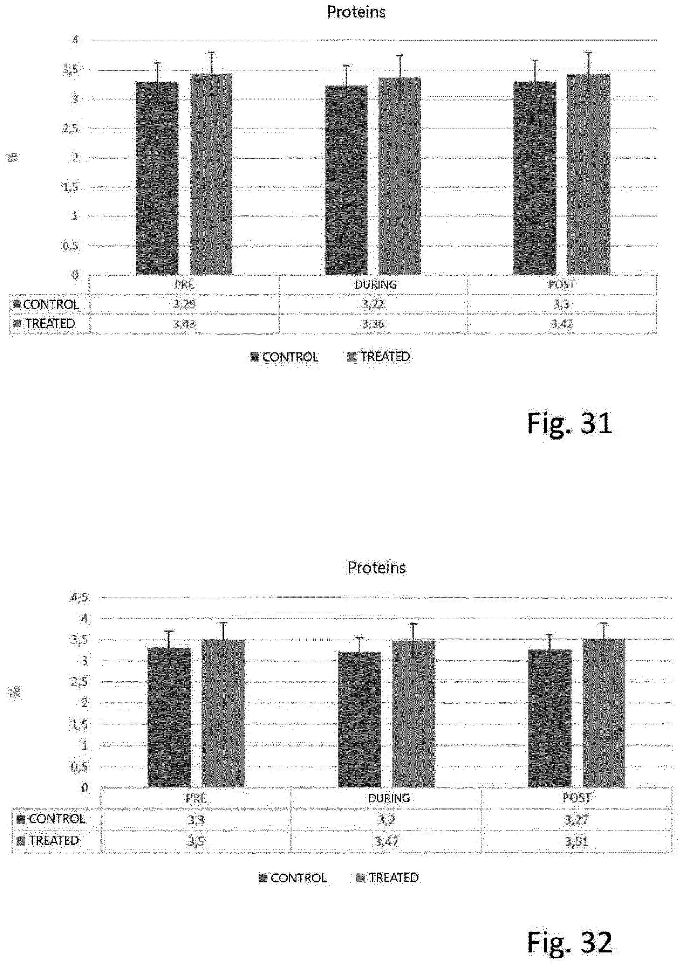

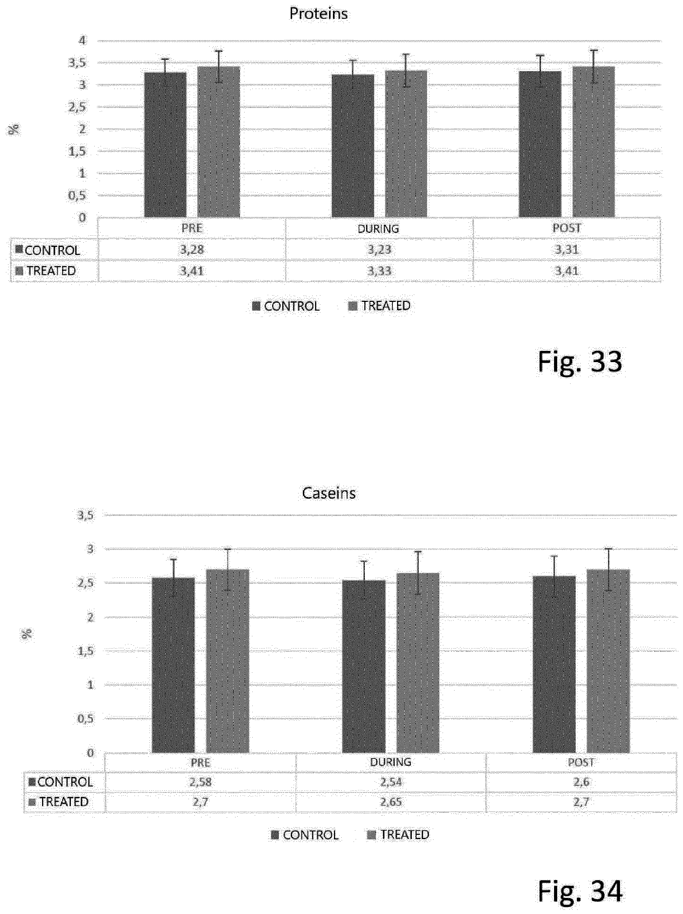

As shown in Figure 31, the data relating to the set of analyses broken down by period of treatment show a similar trend in the treated and control groups, with higher means in the treated group compared to the control group. The values were substantially stable in both groups. When the data were analysed based on state of health, it was possible to observe trend wholly similar to the general one as regards both the presence or absence of treatment and the presence or absence of subclinical mastitis (Figures 32 and 33).

### 4.2.10 Percentage content of caseins

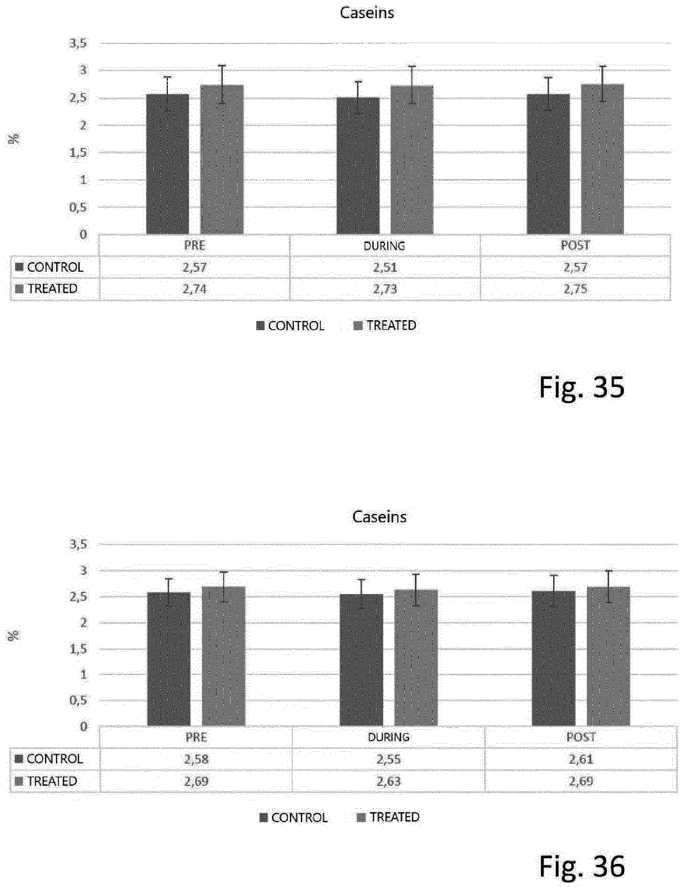

As shown in figure 34, the data relating to the set of analyses broken down by period of treatment show a similar trend in the treated and control groups, with higher means in the treated group compared to the control group. For both groups a slight decrease in the values was observed during the treatment period. When the data were analysed based on state of health, it was possible to observe a trend wholly similar to the general one as regards both the presence or absence of treatment and the presence or absence of subclinical mastitis (Figures 35 and 36).

### 5. Conclusions

The results of the analyses conducted regarding the inflammatory, immune and production parameters have demonstrated that the administration of the composition of the present invention significantly increased the PMN and lymphocyte content in terms of proportion, concentration per ml and total content, compared to the control product, in the treatment period. These results can be defined as scientifically solid, as the statistical analyses took account of the different factors that can influence those parameters, such as the characteristics of the animal and, above all, the state of health of the udder. Moreover, the increase in the amount of PMNs and lymphocytes was not obtained thanks to a significant increase in somatic cells, suggesting an immunomodulating effect, and not only an immunostimulating one. In subjects with subclinical mastitis, in fact, no further increase was observed, which would not be desirable, but rather, in general, a slight decrease was observed compared to the control animals. This aspect is particularly important because in this manner, in addition to the positive effects as regards the physiopathology of the udder, there are no negative effects on the milk hygiene parameters and there are no potential negative effects on milk quality for dairy and profitability purposes. Similarly, the absence of negative qualitative or quantitative effects on the milk demonstrates that the responses obtained are not affected by problems tied to the particular characteristics of the milk.

In conclusion, the composition of the present invention is capable of modulating the immune response by increasing the total number of PMNs and lymphocytes in milk, which represent the main nonspecific cell defences of the udder without giving negative effects to the hygienic quality of the milk (total cell content) and the qualitative characteristics of the milk itself. This effect was observed markedly during the treatment period and, less markedly, in the subsequent post-treatment period.

**Claims**

1. A composition comprising a plant belonging to the genus Ocimum or a portion thereof and/or an extract of a plant belonging to the genus Ocimum, at least one sesquiterpene, at least one vitamin and a plant or a portion thereof belonging to the genus Moringa.

2. The composition according to claim 1, wherein the plant belonging to the genus Ocimum belongs to the species *O.sanctum.*

3. The composition according to claim 1 or 2, wherein the plant belonging to the genus Ocimum is present in a concentration of between 70 and 98% weight/weight (w/w) relative to the weight of the composition, preferably between 80 and 96% w/w, even more preferably between 85 and 95% w/w.

4. The composition according to any one of claims 1-3, wherein the extract of the plant belonging to the genus Ocimum is present in a concentration of between 0.3 and 1.5% volume/weight (v/w) relative to the weight of the composition, preferably between 0.4 and 1.2% v/w, even more preferably between 0.5 and 1% v/w.

5. The composition according to any one of claims 1-4, wherein the extract of the at least one plant belonging to the genus Ocimum has an estragole content of between $2*10^4$ and $7*10^4$ mg/Kg, preferably between $3.5*10^4$ and $5.5*10^4$ mg/Kg relative to the weight of the extract of the at least one plant belonging to the genus Ocimum.

6. The composition according to any one of claims 1-5, wherein the extract of the at least one plant belonging to the genus Ocimum has a 3-carene content of between $1.5*10^4$ and $3.5*10^4$ mg/Kg, preferably between $2*10^4$ and $3*10^4$ mg/Kg relative to the weight of the extract of the at least one plant belonging to the genus Ocimum.

7. The composition according to any one of claims 1-6, wherein the at least one sesquiterpene is β-caryophyllene.

8. The composition according to any one of claims 1-7, wherein the at least one sesquiterpene is present in a concentration of between 0.3 and 1.5% volume/weight (v/w) relative to the weight of the composition, preferably between 0.4 and 1.2% v/w, even more preferably between 0.5 and 1% v/w.

9. The composition according to any one of claims 1-8, wherein the at least one vitamin is present in a concentration of between 0.2 and 1.5% weight/weight (w/w) relative to the weight of the composition, preferably between 0.3 and 1.2 % w/w, even more preferably between 0.4 and 1% w/w.

10. The composition according to any one of claims 1-9, wherein the at least one plant belonging to the genus Moringa is present in a concentration of between 5 and 13% weight/weight (w/w) relative to the weight of the composition, preferably between 6 and 12% w/w, even more preferably between 7 and 10% w/w.

11. The composition according to any one of claims 1-10, formulated as a powder, preferably in the form of a dried or dehydrated powder.

12. The composition according to any one of claims 1-11, for use in the prevention or treatment of a mastitis in a dairy animal.

13. The composition for use according to claim 12, wherein the somatic cell count in the milk obtained from the dairy animal is less than $2*10^5$ cells/ml of milk.

14. The composition for use according to claim 12, wherein the somatic cell count in the milk obtained from the dairy animal is greater than $2*10^5$ cells/ml of milk, preferably greater than $2.5*10^5$ cells/ml of milk.

Fig. 1

Fig. 2

Fig. 3

SCC/ml

Fig. 4

Fig. 5

Fig. 6

Milkings/day

| | PRE | DURING | POST |
|---|---|---|---|
| ■ CONTROL | 2,76 | 2,82 | 2,69 |
| ■ TREATED | 3,06 | 3,07 | 2,91 |

■ CONTROL  ■ TREATED

# Fig. 7

Milkings/day

| | PRE | DURING | POST |
|---|---|---|---|
| ■ CONTROL | 2,76 | 2,55 | 2,4 |
| ■ TREATED | 2,63 | 2,6 | 2,46 |

■ CONTROL  ■ TREATED

# Fig. 8

Fig. 9

Fig. 10

## DSCC/ml

| | PRE | DURING | POST |
|---|---|---|---|
| CONTROL | 79,81 | 75,68 | 78,23 |
| TREATED | 83,67 | 82,72 | 83,1 |

■ CONTROL  ■ TREATED

Fig. 11

## DSCC/ml

| | PRE | DURING | POST |
|---|---|---|---|
| CONTROL | 54,93 | 56,17 | 58,97 |
| TREATED | 58,19 | 64,67 | 62,86 |

■ CONTROL  ■ TREATED

Fig. 12

Fig. 13

Fig. 14

PMN+LYM/ml

| | PRE | DURING | POST |
|---|---|---|---|
| CONTROL | 4,55 | 4,45 | 4,56 |
| TREATED | 4,69 | 4,66 | 4,72 |

CONTROL    TREATED

Fig. 15

PMN+LYM/ml

| | PRE | DURING | POST |
|---|---|---|---|
| CONTROL | 5,69 | 5,3 | 5,39 |
| TREATED | 5,91 | 5,61 | 5,73 |

CONTROL    TREATED

Fig. 16

## PMN+LYM/ml

| | PRE | DURING | POST |
|---|---|---|---|
| CONTROL | 4,22 | 4,21 | 4,34 |
| TREATED | 4,34 | 4,42 | 4,48 |

CONTROL    TREATED

Fig. 17

control    treated

Fig. 18

Fig. 19

Total PMN+LYM

| | PRE | DURING | POST |
|---|---|---|---|
| CONTROL | 9,43 | 9,33 | 9,43 |
| TREATED | 9,53 | 9,44 | 9,52 |

CONTROL    TREATED

Fig. 20

## Total PMN+LYM

| | PRE | DURING | POST |
|---|---|---|---|
| ■ CONTROL | 10,38 | 10,04 | 10,12 |
| ■ TREATED | 10,57 | 10,26 | 10,39 |

■ CONTROL    ■ TREATED

Fig. 21

## Total PMN+LYM

| | PRE | DURING | POST |
|---|---|---|---|
| ■ CONTROL | 9,16 | 9,13 | 9,24 |
| ■ TREATED | 9,23 | 9,24 | 9,32 |

■ CONTROL    ■ TREATED

Fig. 22

Fig. 23

Fig. 24

## Milk production

| | PRE | DURING | POST |
|---|---|---|---|
| CONTROL | 45,56 | 44,98 | 46,25 |
| TREATED | 43,64 | 42,09 | 42,93 |

CONTROL   TREATED

Fig. 25

## Milk production

| | PRE | DURING | POST |
|---|---|---|---|
| CONTROL | 41,11 | 42,03 | 43,21 |
| TREATED | 39,34 | 38,4 | 39,51 |

CONTROL   TREATED

Fig. 26

## Milk production

| | PRE | DURING | POST |
|---|---|---|---|
| CONTROL | 46,85 | 45,8 | 47,07 |
| TREATED | 44,85 | 43 | 43,03 |

CONTROL    TREATED

# Fig. 27

## Fat

| | PRE | DURING | POST |
|---|---|---|---|
| CONTROL | 3,73 | 3,65 | 3,63 |
| TREATED | 3,91 | 3,79 | 3,83 |

CONTROL    TREATED

# Fig. 28

Fat

| | PRE | DURING | POST |
|---|---|---|---|
| ■ CONTROL | 3,85 | 3,64 | 3,76 |
| ■ TREATED | 4,25 | 3,96 | 4,03 |

■ CONTROL    ■ TREATED

Fig. 29

Fat

| | PRE | DURING | POST |
|---|---|---|---|
| ■ CONTROL | 3,7 | 3,65 | 3,6 |
| ■ TREATED | 3,82 | 3,75 | 3,79 |

■ CONTROL    ■ TREATED

Fig. 30

Proteins

| | PRE | DURING | POST |
|---|---|---|---|
| CONTROL | 3,29 | 3,22 | 3,3 |
| TREATED | 3,43 | 3,36 | 3,42 |

■ CONTROL   ■ TREATED

Fig. 31

Proteins

| | PRE | DURING | POST |
|---|---|---|---|
| CONTROL | 3,3 | 3,2 | 3,27 |
| TREATED | 3,5 | 3,47 | 3,51 |

■ CONTROL   ■ TREATED

Fig. 32

## Proteins

| | PRE | DURING | POST |
|---|---|---|---|
| ■CONTROL | 3,28 | 3,23 | 3,31 |
| ■TREATED | 3,41 | 3,33 | 3,41 |

■ CONTROL   ■ TREATED

# Fig. 33

## Caseins

| | PRE | DURING | POST |
|---|---|---|---|
| ■CONTROL | 2,58 | 2,54 | 2,6 |
| ■TREATED | 2,7 | 2,65 | 2,7 |

■ CONTROL   ■ TREATED

# Fig. 34

Caseins

| | PRE | DURING | POST |
|---|---|---|---|
| CONTROL | 2,57 | 2,51 | 2,57 |
| TREATED | 2,74 | 2,73 | 2,75 |

CONTROL    TREATED

Fig. 35

Caseins

| | PRE | DURING | POST |
|---|---|---|---|
| CONTROL | 2,58 | 2,55 | 2,61 |
| TREATED | 2,69 | 2,63 | 2,69 |

CONTROL    TREATED

Fig. 36

# EP 4 382 114 A1

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MUSHTAQ SALEEM ET AL: "Bovine mastitis: An appraisal of its alternative herbal cure", MICROBIAL PATHOGENESIS, ACADEMIC PRESS LIMITED, NEW YORK, NY, US, vol. 114, 9 December 2017 (2017-12-09), pages 357-361, XP085349481, ISSN: 0882-4010, DOI: 10.1016/J.MICPATH.2017.12.024 * page 358, column 2, paragraph 5 – page 359, column 2, paragraph 4 * | 1-14 | INV. A61K36/53 A61K36/185 A61K31/015 A61K31/07 A61K31/355 A61K31/593 A61P29/00 A61P31/00 |
| X | WEI NEE CHENG ET AL: "Moringa Extract Attenuates Inflammatory Responses and Increases Gene Expression of Casein in Bovine Mammary Epithelial Cells", ANIMALS , vol. 9, no. 7 26 June 2019 (2019-06-26), page 391, XP055972687, DOI: 10.3390/ani9070391 Retrieved from the Internet: URL:https://www.ncbi.nlm.nih.gov/pmc/articles/PMC6680921/pdf/animals-09-00391.pdf [retrieved on 2023-06-15] * discussion – conclusion * | 1-14 | |
| | | | TECHNICAL FIELDS SEARCHED (IPC) A61K A61P |
| X | CN 110 051 815 A (WUHAN LONZON BIOMEDICAL TECH CO LTD) 26 July 2019 (2019-07-26) * Summary; claim 4 * | 1,5-11 | |
| X | CN 105 779 218 A (QUANJIAO JINGFU SELENIUM-RICH ECOLOGICAL ANIMAL HUSBANDRY CO LTD) 20 July 2016 (2016-07-20) * claims 1-4 * | 1,4-9,11 | |

-/--

The present search report has been drawn up for all claims

| Place of search | Date of completion of the search | Examiner |
|---|---|---|
| Munich | 15 April 2024 | Markopoulos, Eytyxia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

## EUROPEAN SEARCH REPORT

Application Number

EP 23 21 3930

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 103 535 434 A (HEFEI KANGLING HEALTH PRESERVING SCIENCE & TECHNOLOGY CO LTD) 29 January 2014 (2014-01-29) * claim 1 * | 1,4-9,11 | |
| A | Goretti Maria ET AL: "Composition of essential oils from three Ocimum species obtained by steam and microwave distillation and supercritical CO 2 extraction", Otto Gottlieb ARKIVOC, 2 August 2004 (2004-08-02), pages 66-71, XP093054827, Retrieved from the Internet: URL:https://www.arkat-usa.org/get-file/197 88/ [retrieved on 2023-06-15] * the whole document * | 1-14 | |

TECHNICAL FIELDS
SEARCHED     (IPC)

The present search report has been drawn up for all claims

| Place of search | Date of completion of the search | Examiner |
|---|---|---|
| Munich | 15 April 2024 | Markopoulos, Eytyxia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 3930

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2024

| Patent document cited in search report | Publication date | Patent family member(s) | Publication date |
|---|---|---|---|
| CN 110051815 A | 26-07-2019 | NONE | |
| CN 105779218 A | 20-07-2016 | NONE | |
| CN 103535434 A | 29-01-2014 | NONE | |

EPO FORM P0459

For more details about this annex : see Official Journal of the European Patent Office, No. 12/82